(19) Europäisches Patentamt
European Patent Office
Office européen des brevets

(11) Veröffentlichungsnummer: **0 144 748**
**A2**

(12) **EUROPÄISCHE PATENTANMELDUNG**

(21) Anmeldenummer: **84113259.0**

(22) Anmeldetag: **03.11.84**

(51) Int. Cl.[4]: **C 07 D 233/70**
C 07 D 235/02, C 07 D 401/04
A 01 N 43/50

(30) Priorität: **10.11.83 DE 3340595**

(43) Veröffentlichungstag der Anmeldung:
**19.06.85 Patentblatt 85/25**

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**Hans-Fischer-Strasse 6**
**D-8906 Gersthofen(DE)**

(72) Erfinder: **Handte, Reinhard, Dr.**
**Theilweg 23**
**D-8901 Gablingen(DE)**

(72) Erfinder: **Liebl, Rainer, Dr.**
**An der Weinleite 5b**
**D-8901 Todtenweis(DE)**

(72) Erfinder: **Mildenberger, Hilmar, Dr.**
**Fasanenstrasse 24**
**D-6233 Kelkheim (Taunus)(DE)**

(72) Erfinder: **Bauer, Klaus, Dr.**
**Kolpingstrasse 7**
**D-6054 Rodgau(DE)**

(72) Erfinder: **Bieringer, Hermann, Dr.**
**Eichenweg 26**
**D-6239 Eppstein/Taunus(DE)**

(72) Erfinder: **Bürstell, Helmut, Dr.**
**Am Hohlacker 65**
**D-6000 Frankfurt am Main 50(DE)**

(54) **Imidazolinone, Verfahren zur ihrer Herstellung und ihre Verwendung im Pflanzenschutz.**

(57) Imidazolinone der Formel

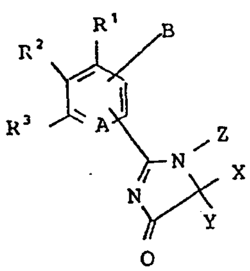

I

worin A N oder C-$R^4$; B Halogenalkyl, Alkoxymethyl, Cyanmethyl oder Thiocyanatomethyl; X Alkyl; Y Alkyl, Cycloalkyl, Alkenyl, Phenyl oder Benzyl; oder X und Y zusammen mit C eine Spiro-cycloalkylgruppe; Z H, (subst.) Alkyl, Alkenyl, Propargyl, eine Alkyl-, Carbonester- oder Sulfoestergruppe; $R^1$-$R^4$ H, Halogen, Alkyl, Alkoxy, Alkoxycarbonyl, Halogenalkyl, $NO_2$, CN, (subst.) Phenoxy, (subst.) Phenyl oder je zwei benachbarte zusammen den Rest $-CH=CH-CH-CH-$ bedeuten, sind wirksame Herbizide und Wachstumsregulatoren.

EP 0 144 748 A2

Croydon Printing Company Ltd.

0144748

HOECHST AKTIENGESELLSCHAFT HOE 83/F 233 Dr.AU/mk

Imidazolinone, Verfahren zu ihrer Herstellung und ihre Verwendung im Pflanzenschutz

Die vorliegende Erfindung betrifft neue, herbizid wirksame Imidazolinone der Formel (I)

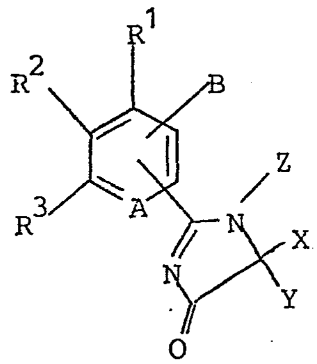

(I)

in welcher

A N oder $C-R^4$;

B Halogen-$(C_1-C_2)$alkyl wobei unter Halogen Fluor, Chlor und Brom, vorzugsweise Fluor und Chlor zu verstehen sind, $(C_1-C_4)$ Alkoxymethyl, Cyanmethyl und Thiocyanatomethyl;

X $(C_1-C_4)$Alkyl;

Y $(C_1-C_6)$Alkyl, Cyclo$(C_3-C_6)$alkyl, $(C_2-C_4)$Alkenyl, $(C_2-C_4)$Alkinyl, Phenyl oder Benzyl;

X und Y zusammen mit dem Kohlenstoffatom, an das sie gebunden sind, eine gegebenenfalls durch $-CH_3$ substituierte Spirocyclo$(C_3-C_6)$alkylgruppe;

Z Wasserstoff, $(C_1-C_4)$Alkyl, welches durch $(C_1-C_4)$Alkoxycarbonyl substituiert sein kann, $(C_3-C_4)$Alkenyl, Propargyl, $-CO-R^5$ oder $-SO_2-R^6$;

$R^1$, $R^2$, $R^3$ und $R^4$ unabhängig voneinander Wasserstoff,

Halogen, $(C_1-C_4)$Alkyl, $(C_1-C_6)$Alkoxy, $(C_1-C_6)$ Alkoxycarbonyl, Halogen-$(C_1-C_2)$alkyl, Nitro, Cyano, Phenoxy und Phenyl, das gegebenenfalls mit $(C_1-C_4)$Alkyl, $(C_1-C_4)$Alkoxy oder Halogen substituiert sein kann, wobei jeweils zwei o-ständige Reste $R^1$, $R^2$, $R^3$ oder $R^4$ auch gemeinsam die Gruppierung -CH=CH-CH=CH bilden können;

$R^5$ $(C_1-C_{12})$Alkyl, das gegebenenfalls mit bis zu zwei $(C_1-C_4)$Alkoxygruppen oder mit bis zu drei Halogen substituiert ist; Phenyl, das mit bis zu zwei Halogenatomen, einer Methyl-, einer Nitro- oder einer Methoxygruppe substituiert sein kann; Cyclo$(C_3-C_7)$alkyl, $(C_1-C_4)$Alkoxy, $(C_1-C_4)$Alkoxycarbonyl, Benzyloxy, Phenoxy oder $-NR^7R^8$, insbesondere $(C_1-C_{12})$Alkyl;

$R^6$ $(C_1-C_4)$Alkyl, $CF_3$, $CCl_3$, Phenyl, Chlorphenyl oder Methylphenyl;

$R^7$ Wasserstoff oder $(C_1-C_4)$Alkyl; und

$R^8$ $(C_1-C_4)$Alkyl, Phenyl, Chlorphenyl, Methylphenyl, Amino, Mono- oder Di$(C_1-C_4)$alkylamino, bedeuten,

sowie deren optischen Isomere (falls $X \neq Y$), ihre Säureadditionssalze und N-Oxide (für den Fall, daß A für N steht).

Man erhält die erfindungsgemäßen Verbindungen, indem man die Amide der Formel (II)

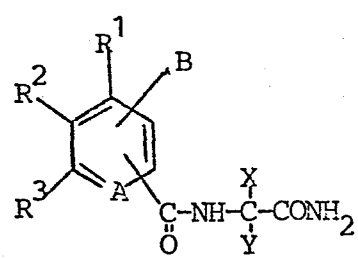

(II)

durch Wasserabspaltung cyclisiert und gewünschtenfalls die erhaltenen Verbindungen mit Z = Wasserstoff

durch Alkylierung, Acylierung, Sulfonierung, Oxidation oder Salzbildung in andere Verbindungen der Formel (I) überführt.

Die Cyclisierung der Amide (II) kann z.B. mit Phosphorpentachlorid, vorteilhaft in Gegenwart eines unter den Reaktionsbedingungen inerten Lösemittels erfolgen. Als Beispiele für letztere seien genannt: Toluol, Xylol, Chloroform oder Phosphoroxychlorid. Die Reaktionstemperatur ist unkritisch und kann zwischen -10°C und +150°C variiert werden. Besonders vorteilhaft sind Reaktionstemperaturen zwischen 0 und 100°C. Man erhält dabei primär die Hydrochloride der Imidazolinone (I). Nach üblichen Methoden, z.B. durch Umsetzung mit Natriumcarbonat oder Natriumhydrogencarbonat, lassen sich daraus die freien Basen herstellen.

Die Cyclisierung kann ebenfalls in Gegenwart starker organischer oder anorganischer Säuren, wie Schwefelsäure oder p-Toluolsulfonsäure, unter gleichzeitiger Abtrennung des gebildeten Wassers bei Temperaturen von 0°C bis 150°C erfolgen. Besonders vorteilhaft läßt sich die Reaktion so führen, daß das gebildete Wasser durch Azeotropdestillation mit einem Lösemittel, wie Toluol, Xylol oder Chloroform, abgetrennt wird. Nach Neutralisation können die Produkte nach üblichen Methoden isoliert werden.

Die Imidazolinone der Formel (I) (Z = Wasserstoff) werden dabei im allgemeinen in guten Ausbeuten erhalten. Dies war für die halogenalkylsubstituierten Vertreter (B = Halogenalkyl) überraschend, da vielmehr angenommen werden mußte, daß sich die Imidazolinone (I), auf Grund der sterisch sehr günstigen Anordnung der Halogenalkylgruppe, zum basischen Imidazolinstickstoff unter Halogenwasserstoff

abspaltung zersetzen würden. Die Cyclisierungsprodukte der Formel (I) mit Z = Wasserstoff können in einfacher Weise nach an sich bekannten Verfahren mit Alkylierungsmitteln, (Methyljodid, Dimethylsulfat) oder Acylierungsmitteln (wie Säurechloriden) in Gegenwart von Basen, oder mit Isocyanaten umgesetzt werden.

Die erfindungsgemäßen Verbindungen sind, wenn Z für Wasserstoff steht, tautomer, so daß sie in einer der beiden Formen (I a)/(I b) oder als Gemisch von (I a) und (I b) vorliegen können. Diese Isomeren treten auch bei den Derivaten mit Z ‡ H auf.

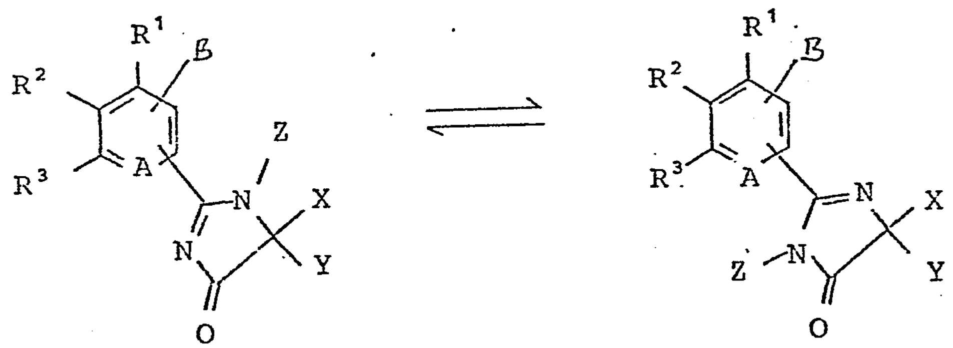

(I a) (I b)

Die Definitionen der Formel (I) umfassen stets beide isomeren Strukturen der Formel (I a) und (I b).

Sowohl die Säureadditionssalze, als auch die N-Oxide der Verbindungen der Formel (I) sind auf allgemein bekanntem Weg gut zugänglich, letztere z.B. durch Umsetzung mit Peroxiden oder $H_2O_2$.

Die Amide der Formel (II) lassen sich leicht aus den Aminoamiden (III) und den entsprechend substituierten Carbonsäurederivaten (IV) erhalten. Als Derivate geeignet sind z.B. Säurechloride oder Kohlensäurealkylester ($R^9$ = Cl, -O-COO-Alkyl).

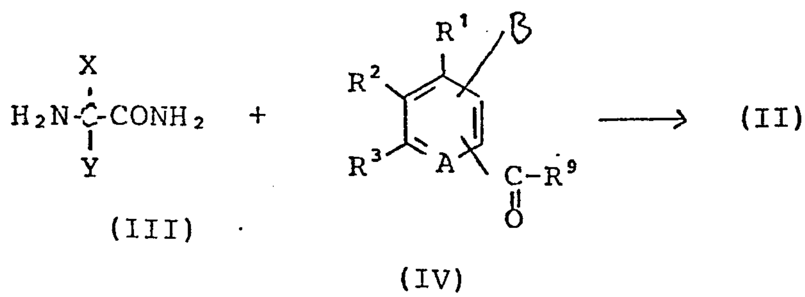

Die vorliegenden erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Wurzelunkräuter werden durch die Wirkstoffe gut erfaßt. Dabei ist es gleichgültig, ob die Substanzen in Vorsaat-, Vorauflauf- oder Nachauflaufspritzung ausgebracht werden. Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dann ihr Wachstum ein und sterben schließlich nach 3 Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann. Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar

...

nicht geschädigt. Die erfindungsgemäßen Substanzen besitzen somit ausgezeichnete Selektivität bei Kulturpflanzen und eignen sich aus diesen Gründen sehr gut zur Bekämpfung von unerwünschtem Pflanzenwuchs in landwirtschaftlichen Nutzungspflanzungen.

Darüberhinaus weisen sie wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abzession und Wuchsstauchung eingesetzt werden. Desweiteren eignen sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikolylen Kulturen eine große Rolle, da das Lagern hierdurch verringert oder völlig verhindert werden kann.

Gegenstand der vorliegenden Erfindung sind daher auch herbizide und wachstumsregulatorische Mittel, welche den Wirkstoff der Formel (I) neben üblichen Formulierungshilfsmitteln enthalten.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden. Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkyl-arylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit fein verteilten, festen Stoffen, z. B. Talkum,

natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte

...

Zubereitungen sowie versprühbare Lösungen werden vor der Anwendung üblicherweise nicht mehr mit weiteren inerten Stoffen verdünnt.

Die Aufandmengen an Wirkstoff der Formel (I) variieren je nach Indikation zwischen 0,01 und 10 kg Wirkstoff/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt:

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (z.B. (R)Triton X 207 der Rohm & Haas Co.), 3 Gew.-Tl. Isotridecanol polyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon

als Lösungsmittel und 10 Gewichtsteilen oxethyliertes Nonylphenol (10 AeO) als Emulgator.

Die folgenden Beispiele dienen der weiteren Erläuterung der Erfindung:

A. Herstellungsbeispiele

Beispiel 1 Ausgangsstoff

2-Trifluormethylbenzoesäure-N-2-(2-carbamoyl-3-methyl) butylamid

Zu einer Lösung aus 10,3g(0,079 mol) 2-Methylvalinamid und 8,7 g (0,086 mol) Triethylamin in 50 ml abs. Methylenchlorid tropft man bei 0 - 5°C 15 g (0,072 mol) 2-Trifluormethylbenzoylchlorid zu. Nach 1 h Reaktionszeit bei Raumtemperatur wird auf 500 ml 0,5 n Natriumhydrogencarbonatlösung gegossen, nachgerührt und abgesaugt. Nach dem Trocknen erhält man 18,3 g (85 % der Theorie) 2-Trifluormethylbenzoesäure-N-2-(2-carbamoyl-3-methyl)-butylamid als farbloser Feststoff vom Fp. 124 bis 126°C.

Beispiel 2

5-Isopropyl-5-methyl-4-oxo-2-(2-trifluormethylphenyl)-2-imidazolin

18,3 g (0,060 mol) 2-Trifluormethylbenzoesäure-N-2-(2-carbamoyl-3-methyl)-butylamid werden in 100 ml Phosphoroxychlorid gelöst und bei Raumtemperatur portionsweise mit 14,0 g (0,067 mol) Phosphorpentachlorid versetzt. Nach 5 h bei Raumtemperatur wird im Vakuum eingedampft, mit Eis hydrolysiert, mit Natriumhydrogencarbonat neutralisiert und die Lösung 2mal mit je 100 ml Essigester extrahiert. Nach dem Trocknen über Natriumsulfat und

Eindampfen erhält man 15,5 g (91 % der Theorie) 5-Isopropyl-5-methyl-4-oxo-2-(2-trifluormethylphenyl)-2-imidazolin als farblosen Feststoff vom Fp. 109 bis 110°C.

Beispiel 3

2-(2-Dichlormethylphenyl)-5-5-diethyl-4-oxo-1-(4-toluolsulfonyl)-2-imidazolin

Zu 4,5 g (0,015 mol) 2-(2-Dichlormethylphenyl)-5-5-diethyl-4-oxo-2-imidazolin in 30 ml abs. Pyridin gibt man bei 0 - 5°C 3,2 g (0,017 mol) 4-Toluolsulfonsäurechlorid zu, läßt 1 d bei Raumtemperatur stehen, gießt auf 500 ml Wasser, extrahiert 2mal mit je 100 ml Toluol, wäscht die org. Phase 1mal mit 2 n Natronlauge und 1mal mit Wasser. Nach dem Trocknen (Natriumsulfat) und Ein-Eindampfen wird der Rückstand chromatographisch (Kieselgel, LM Petrolether-Essigester 7 : 3) gereinigt. Man erhält 4,4 g (66 % der Theorie) 2-(2-Dichlormethylphenyl)-5-diethyl-4-oxo-1-(4-toluolsulfonyl)-2-imidazolin als farbloser Feststoff vom Fp. 139 - 140°C.

Tabelle 1

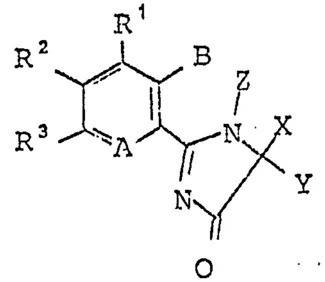

| Bei-spiel | A | B | X | Y | Z | $R^1$ | $R^2$ | $R^3$ | $R^4$ | Fp [°C] |
|---|---|---|---|---|---|---|---|---|---|---|
| 4 | $C-R^4$ | $-CH_2Cl$ | $-CH_3$ | $-CH(CH_3)_2$ | H | H | H | H | H | Oel |
| 5 | " | " | " | " | " | " | " | " | " | 167 (Hydro-chlorid) |
| 6 | " | $-CHCl-CH_3$ | " | $-C_2H_5$ | " | " | " | " | " | Oel |
| 7 | " | $-CHCl_2$ | " | $-CH_3$ | " | " | " | " | " | 156-7 |
| 8 | " | " | " | $-C_2H_5$ | " | " | " | " | " | 145-8 |
| 9 | " | " | " | $-CH(CH_3)_2$ | " | " | " | " | " | 147-9 |
| 10 | " | " | " | " | " | " | " | " | " | 185-7 (Hydro-chlorid) |
| 11 | " | " | " | " | $-COCH_3$ | " | " | " | " | Harz |
| 12 | " | " | " | " | $-CONHCH_3$ | " | " | " | " | Oel |
| 13 | " | " | " | " | $-COCH(CH_3)_2$ | " | " | " | " | " |
| 14 | " | " | " | " | -CO-Phenyl | " | " | " | " | " |
| 15 | " | " | " | " | $-SO_2-CH_3$ | " | " | " | " | Harz |
| 16 | " | " | " | " | $-COOC_2H_5$ | " | " | " | " | 93 |
| 17 | " | " | " | " | H | " | " | Cl | " | 138-42 |

...

Fortsetzung Tabelle 1

| Bei-spiel | A | B | X | Y | Z | $R^1$ | $R^2$ | $R^3$ | $R^4$ | Fp [°C] |
|---|---|---|---|---|---|---|---|---|---|---|
| 18 | $C-R^4$ | $-CHCl_2$ | $-CH_3$ | $-CH(CH_3)_2$ | H | Cl | H | Cl | H | 140-3 |
| 19 | " | " | " | " | " | H | $-CHCl_2$ | H | " | 135 (Zers.) |
| 20 | N | " | " | " | " | " | " | " | - | Oel |
| 21 | " | " | " | " | $-COCH_3$ | " | " | " | - | " |
| 22 | $C-R^4$ | " | " | $-CH_2-CH(CH_3)_2$ | H | " | " | " | " | 122-3 |
| 23 | " | " | $-C_2H_5$ | $-C_2H_5$ | " | " | " | " | " | 142 |
| 24 | " | " | $-CH(CH_3)-CH_2-CH_2-CH_2-CH_2-$ | | " | " | " | " | " | Oel |
| 25 | " | " | $-CH_3$ | $-CH(CH_3)_2$ | " | " | " | $-CH=CH-CH=CH-$ | | " |
| 26 | N | " | " | " | " | " | $-CH=CH-CH=CH-$ | | - | " |
| 27 | $C-R^4$ | $-CCl_3$ | " | " | " | " | H | H | H | 145 (Zers.) |
| 28 | " | " | " | " | $CH(CH_3)COOCH_3$ | | | | " | |
| 29 | " | " | " | " | $COCOOCH_3$ | | | | " | |
| 30 | " | " | " | Cyclopropyl | $CONHN(CH_3)_2$ | | | | " | |
| 31 | " | $CHCl_2$ | " | $-CH(CH_3)_2$ | $CH_3$ | | | | " | Öl |
| 32 | " | " | " | " | $CH_2COOCH_3$ | | | | " | Öl |
| 33 | " | " | " | " | $CH(CH_3)COOC_2H_5$ | | | | " | Öl |
| 34 | " | $CHOCH_3$ | " | " | " | | | | " | 70-74 |

B. Biologische Beispiele

Prüfung auf herbizide Wirkung

Die Schädigung der Unkrautpflanzen bzw. die Kulturpflanzenverträglichkeit wurde in einem Schlüssel von 0 - 5 bonitiert.
Dabei bedeutet

0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

1. Wirkung gegen Unkräuter

Samen bzw. Rhizomstücke mono- und dikotyler Unkräuter wurden in Lehmerde in Plastiktöpfen (Ø 9 cm) ausgelegt und mit Erde abgedeckt. Die als benetzbare Pulver bzw. als Emulsionskonzentrate formulierten erfindungsgemäßen Verbindungen wurden in Form wäßriger Suspensionen bzw. Emulsionen auf die Erdoberfläche appliziert. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur: 23 ± 1 °C; rel. Luftfeuchte 60 - 80 %) kultiviert. Nach ca. 3 Wochen wurde die Pflanzenschädigung visuell bonitiert. Als Vergleich dienten dabei unbehandelte Kontrollen.
Wie aus den Werten der Tabelle 2 hervorgeht, weisen

die erfindungsgemäßen Verbindungen im Vorauflaufverfahren eine zum Teil ausgezeichnete herbizide Wirksamkeit gegen wirtschaftlich bedeutende mono- und dikotyle Schadpflanzen auf.

In ähnlicher Weise wurden verschiedene Unkräuter in Töpfen im Gewächshaus bis zum 3-6-Blattstadium herangezogen und dann im Nachauflaufverfahren mit den erfindungsgemäßen Verbindungen (formuliert als Spritzpulver) behandelt. 4 Wochen später wurden die Versuchspflanzen im Vergleich zu unbehandelten Kontrollpflanzen visuell bonitiert, indem die Schädigung geschätzt wurde.
Die Ergebnisse des Versuchs (Tabelle 3) belegen die guten herbiziden Eigenschaften der Verbindungen.

Tabelle 2
Herbizide Wirksamkeit im Vorauflaufverfahren (% Schädigung)

| Beispiel-Nr. | kg AS/ha | STM | CRS | SIA | LOM | ECG |
|---|---|---|---|---|---|---|
| 4 | 2,4 | 5 | 5 | 5 | 5 | 5 |
| 5 | 2,4 | 5 | 5 | 5 | 5 | 5 |
| 9 | 2,4 | 5 | 5 | 5 | 5 | 5 |
| 10 | 2,4 | 5 | 5 | 5 | 5 | 5 |
| 11 | 2,4 | 5 | 5 | 5 | 5 | 5 |
| 22 | 2,4 | | | | 4 | 4 |
| 23 | 2,4 | 4 | | 5 | 4 | |
| 27 | 2,4 | 5 | 5 | 5 | 5 | 5 |
| 34 | 2,4 | 5 | | 5 | 4 | 4 |

...

Tabelle 2 (Fortsetzung)

| Beispiel Nr. | kg AS/ha | ALM | POA | AMR | SIA | TAW | GS |
|---|---|---|---|---|---|---|---|
| 4 | 2,4 | 5 | - | 5 | 5 | - | - |
| 5 | 2,4 | 5 | - | 5 | 5 | 5 | 5 |
| 7 | 2,4 | 4 | 5 | 5 | 5 | - | - |
| 8 | 2,4 | 5 | 5 | 5 | 5 | - | - |
| 9 | 2,4 | 5 | 5 | 5 | 5 | 5 | 5 |
| 10 | 2,4 | 5 | 5 | 5 | 5 | 5 | 5 |
| 11 | 2,4 | 5 | - | 5 | 5 | 4 | 4 |
| 12 | 2,4 | 5 | 5 | 5 | 5 | 5 | 4 |
| 14 | 2,4 | 5 | 5 | 5 | 5 | 5 | 4 |
| 15 | 2,4 | 5 | - | 5 | 5 | - | - |
| 16 | 2,4 | 4 | 5 | 5 | 5 | 4 | 4 |
| 17 | 2,4 | - | - | 5 | 4 | - | - |
| 27 | 2,4 | 5 | - | 5 | 5 | 5 | 5 |
| 33 | 2,4 | 5 | 5 | 5 | 5 | 5 | 5 |

ALM = Alopecurus myosuroides
POA = Poa annua
AMR = Amaranthus retroflexus
TAW = Triticum aestivum (Winter-)
GS = Glycine soja

STM = Stellaria media
CRS = Chrysanthemum segetum
SIA = Sinapis alba
LOM = Lolium multiflorum
ECG = Echinochloa crus-galli

Tabelle 3

Herbizide Wirksamkeit im Nachauflaufverfahren (% Schädigung)

| Beispiel-Nr. | kg AS/ha | SIA | CRS | ECG | LOM |
|---|---|---|---|---|---|
| 4 | 2,4 | 5 | 5 | 4 | 4 |
| 5 | 2,4 | 5 | 4 | 4 | 4 |
| 9 | 2,4 | 5 | – | – | – |
| 10 | 2,4 | 4 | – | – | – |
| 11 | 2,4 | 5 | – | 4 | 4 |
| 27 | 2,4 | 5 | 5 | 4 | 4 |

Tabelle 3 (Fortsetzung)

| Beispiel Nr. | kg AS/ha | ALM | POA | AMR | SIA | GS |
|---|---|---|---|---|---|---|
| 4 | 2,4 | – | – | 5 | 5 | 5 |
| 5 | 2,4 | 4 | 4 | 5 | 5 | – |
| 9 | 2,4 | – | – | 5 | 5 | – |
| 11 | 2,4 | – | – | 5 | 5 | – |
| 12 | 2,4 | – | – | 5 | 5 | – |
| 14 | 2,4 | – | – | 5 | 5 | – |
| 15 | 2,4 | – | – | 5 | 5 | – |
| 16 | 2,4 | – | – | 5 | 4 | – |
| 18 | 2,4 | – | – | 5 | – | – |
| 27 | 2,4 | – | 5 | 5 | 5 | – |
| 33 | 2,4 | – | – | 5 | 5 | – |

Prüfung auf wachstumsregulierende Wirkung

1. Wuchshemmung bei Getreide

In Schalenversuchen im Gewächshaus wurden junge Getreidepflanzen (Weizen, Gerste und Roggen) im 3-Blattstadium mit den zu prüfenden Verbindungen in den in Tabelle 4 angegebenen Wirkstoffkonzentrationen (kg/ha) tropfnaß gespritzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in Tabelle 4 zusammengefaßt. Bei der Angabe der Wuchshemmung bedeuten 100 % den Stillstand des Wachstums und 0 % ein Wachstum entsprechend dem der unbehandelten Kontrollpflanzen.

Tabelle 4

| Verbindung n. Beispiel Nr. | Anwendungskonzentration (kg/ha) | Wuchshemmung in % bei | | | Phytotox. Wirkung |
|---|---|---|---|---|---|
| | | Weizen | Gerste | Roggen | |
| 4 | 2,5 | 29 | 31 | 25 | keine |
| | 1,25 | 27 | 29 | 20 | Schäden |
| 10 | 2,5 | 22 | 28 | 27 | geringe |
| | 1,25 | 15 | 24 | 26 | Schäden |
| 27 | 2,5 | 26 | 29 | 22 | keine |
| | 1,25 | 24 | 27 | 19 | Schäden |

2. Wuchshemmung an Buschbohnen

10 - 15 cm Buschbohnen wurden mit den Wirkstoffzubereitungen tropfnaß bespritzt. Nach 2 Wochen wird der Zuwachs gemessen und die Wuchshemmung in % des Zuwachses der Kontrollpflanzen berechnet. Die Ergebnisse sind in Tabelle 5 zusammengefaßt.

Tabelle 5

| Verbindung gemäß Beispiel Nr. | Anwendungskonzentration (kg/ha) | Wuchshemmung in % | Phytotox. Wirkung |
|---|---|---|---|
| 10 | 2,5 | 41 | geringe |
| | 1,25 | 28 | Schäden |
| 27 | 2,5 | 39 | keine |
| | 1,25 | 31 | Schäden |

1. Imidazolinone der Formel (I)

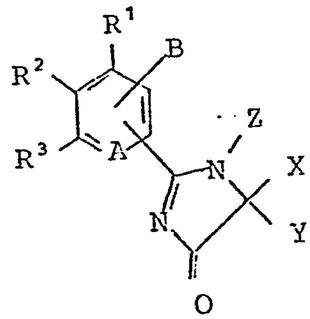

(I)

in welcher

A N oder $C-R^4$;

B Halogen-$(C_1-C_2)$alkyl wobei unter Halogen Fluor, Chlor und Brom, vorzugsweise Fluor und Chlor zu verstehen sind, $(C_1-C_4)$ Alkoxymethyl, Cyanmethyl und Thiocyanatomethyl;

X $(C_1-C_4)$Alkyl;

Y $(C_1-C_6)$Alkyl, Cyclo$(C_3-C_6)$alkyl, $(C_2-C_4)$Alkenyl, $(C_2-C_4)$Alkinyl, Phenyl oder Benzyl;

X und Y zusammen mit dem Kohlenstoffatom, an das sie gebunden sind, eine gegebenenfalls durch $-CH_3$ substituierte Spirocyclo$(C_3-C_6)$alkylgruppe;

Z Wasserstoff, $(C_1-C_4)$Alkyl, welches durch $(C_1-C_4)$Alkoxycarbonyl substituiert sein kann, $(C_3-C_4)$Alkenyl, Propargyl, $-CO-R^5$ oder $-SO_2-R^6$;

$R^1$, $R^2$, $R^3$ und $R^4$ unabhängig voneinander Wasserstoff,

Halogen, $(C_1-C_4)$Alkyl, $(C_1-C_6)$Alkoxy, $(C_1-C_6)$ Alkoxycarbonyl, Halogen-$(C_1-C_2)$alkyl, Nitro, Cyano, Phenoxy und Phenyl, das gegebenenfalls mit $(C_1-C_4)$Alkyl, $(C_1-C_4)$Alkoxy oder Halogen substituiert sein kann, wobei jeweils zwei o-ständige Reste $R^1$, $R^2$, $R^3$ oder $R^4$ auch gemeinsam die Gruppierung -CH=CH-CH=CH bilden können;

$R^5$ $(C_1-C_{12})$Alkyl, das gegebenenfalls mit bis zu zwei $(C_1-C_4)$Alkoxygruppen oder mit bis zu drei Halogen substituiert ist; Phenyl, das mit bis zu zwei Halogenatomen, einer Methyl-, einer Nitro- oder einer Methoxygruppe substituiert sein kann; Cyclo$(C_3-C_7)$alkyl, $(C_1-C_4)$Alkoxy, $(C_1-C_4)$Alkoxycarbonyl, Benzyloxy, Phenoxy oder $-NR^7R^8$, insbesondere $(C_1-C_{12})$Alkyl;

$R^6$ $(C_1-C_4)$Alkyl, $CF_3$, $CCl_3$, Phenyl, Chlorphenyl oder Methylphenyl;

$R^7$ Wasserstoff oder $(C_1-C_4)$Alkyl; und

$R^8$ $(C_1-C_4)$Alkyl, Phenyl, Chlorphenyl, Methylphenyl, Amino, Mono- oder Di$(C_1-C_4)$alkylamino, bedeuten, sowie deren optischen Isomere (falls X $\neq$ Y), ihre Säureadditionssalze und N-Oxide (für den Fall, daß A für N steht).

2. Verfahren zur Herstellung von Verbindungen der Formel (I), dadurch gekennzeichnet, daß man die Amide der Formel (II)

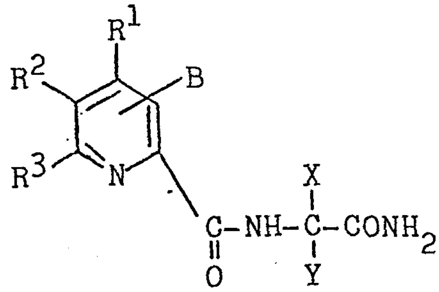

(II)

durch Wasserabspaltung cyclisiert und gewünschtenfalls die erhaltenen Verbindungen mit Z = Wasserstoff

durch Alkylierung, Acylierung, Sulfonierung, Oxidation oder Salzbildung in andere Verbindungen der Formel I überführt.

3. Verwendung der Verbindungen der Formel (I) nach Anspruch 1 als Herbizide und Wachstumsregulatoren.

4. Herbizide und wachstumsregulierende Mittel, dadurch gekennzeichnet, daß sie eine Verbindung der allgemeinen Formel (I) nach Anspruch 1 enthalten.

5. Verfahren zur Bekämpfung von unerwünschten Pflanzen, bzw. zur Wachstumsregulierung, dadurch gekennzeichnet, daß man eine wirksame Menge einer Verbindung der Formel (I) nach Anspruch 1 auf die zu behandelnde Anbaufläche bzw. die zu behandelnden Pflanzen appliziert.

PATENTANSPRÜCHE ÖSTERREICH:

1. Verfahren zur Herstellung von Imidazolinonen der Formel (I)

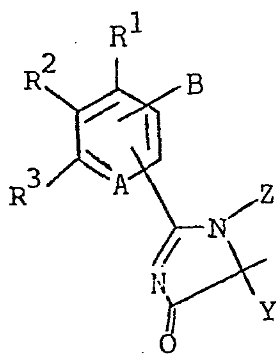

(I)

in welcher

A N oder $C-R^4$;

B Halogen-$(C_1-C_2)$alkyl wobei unter Halogen Fluor, Chlor und Brom, vorzugsweise Fluor und Chlor zu verstehen sind, $(C_1-C_4)$ Alkoxymethyl, Cyanmethyl und Thiocyanatomethyl;

X $(C_1-C_4)$Alkyl;

Y $(C_1-C_6)$ Alkyl, Cyclo$(C_3-C_6)$alkyl, $(C_2-C_4)$Alkenyl, $(C_2-C_4)$Alkinyl, Phenyl oder Benzyl;

X und Y zusammen mit dem Kohlenstoffatom, an das sie gebunden sind, eine gegebenenfalls durch $-CH_3$ substituierte Spirocyclo$(C_3-C_6)$alkylgruppe;

Z Wasserstoff, $(C_1-C_4)$Alkyl, welches durch $(C_1-C_4)$Alkoxycarbonyl substituiert sein kann, $(C_3-C_4)$Alkenyl, Propargyl, $-CO-R^5$ oder $-SO_2-R^6$;

$R^1$, $R^2$, $R^3$ und $R^4$ unabhängig voneinander Wasserstoff,

Halogen, $(C_1-C_4)$Alkyl, $(C_1-C_6)$Alkoxy, $(C_1-C_6)$ Alkoxycarbonyl, Halogen-$(C_1-C_2)$alkyl, Nitro, Cyano, Phenoxy und Phenyl, das gegebenenfalls mit $(C_1-C_4)$Alkyl, $(C_1-C_4)$Alkoxy oder Halogen substituiert sein kann, wobei jeweils zwei o-ständige Reste $R^1$, $R^2$, $R^3$ oder $R^4$ auch gemeinsam die Gruppierung -CH=CH-CH=CH bilden können;

$R^5$ $(C_1-C_{12})$Alkyl, das gegebenenfalls mit bis zu zwei $(C_1-C_4)$Alkoxygruppen oder mit bis zu drei Halogen substituiert ist; Phenyl, das mit bis zu zwei Halogenatomen, einer Methyl-, einer Nitro- oder einer Methoxygruppe substituiert sein kann; Cyclo$(C_3-C_7)$alkyl, $(C_1-C_4)$Alkoxy, $(C_1-C_4)$Alkoxycarbonyl, Benzyloxy, Phenoxy oder $-NR^7R^8$, insbesondere $(C_1-C_{12})$Alkyl;

$R^6$ $(C_1-C_4)$Alkyl, $CF_3$, $CCl_3$, Phenyl, Chlorphenyl oder Methylphenyl;

$R^7$ Wasserstoff oder $(C_1-C_4)$Alkyl; und

$R^8$ $(C_1-C_4)$Alkyl, Phenyl, Chlorphenyl, Methylphenyl, Amino, Mono- oder Di$(C_1-C_4)$alkylamino, bedeuten, sowie deren optischen Isomere (falls $X \neq Y$), ihre Säureadditionssalze und N-Oxide (für den Fall, daß A für N steht), dadurch gekennzeichnet, daß man die Amide der Formel (II)

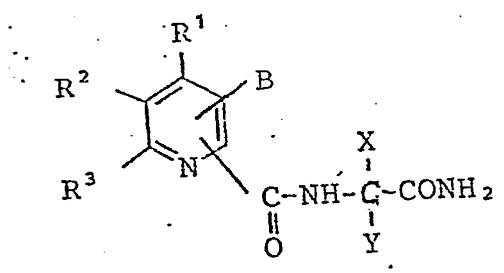

(II)

durch Wasserabspaltung cyclisiert und gewünschtenfalls die erhaltenen Verbindungen mit Z = Wasserstoff

durch Alkylierung, Acylierung, Sulfonierung, Oxidation oder Salzbildung in andere Verbindungen der Formel (I) überführt.

2. Verwendung der Verbindungen der Formel (I) nach Anspruch 1 als Herbizide und Wachstumsregulatoren.

3. Herbizide und wachstumsregulierende Mittel, dadurch gekennzeichnet, daß sie eine Verbindung der allgemeinen Formel (I) nach Anspruch 1 enthalten.

4. Verfahren zur Bekämpfung von unerwünschten Pflanzen, bzw. zur Wachstumsregulierung, dadurch gekennzeichnet, daß man eine wirksame Menge einer Verbindung der Formel (I) nach Anspruch 1 auf die zu behandelnde Anbaufläche bzw. die zu behandelnden Pflanzen appliziert.